# EUROPÄISCHE PATENTSCHRIFT

(11) **EP 1 049 765 B1**
(45) Veröffentlichungstag und Bekanntmachung des Hinweises auf die Patenterteilung: **23.11.2005**
(21) Anmeldenummer: 99903646.0
(22) Anmeldetag: 19.01.1999
(51) Int. Cl.: C12M 1/36, C12M 3/06, C12M 1/12

(54) **KULTURVORRICHTUNG UND VERFAHREN ZUR KULTIVIERUNG VON ZELLEN ODER GEWEBEKOMPONENTEN**
CULTURE DEVICE AND METHOD FOR CULTIVATING CELLS OR TISSUE COMPONENTS
DISPOSITIF DE CULTURE ET PROCEDE DE CULTURE DE CELLULES OU DE COMPOSANTS TISSULAIRES

(30) Priorität: 19.01.1998 DE 19801763; 23.04.1998 US 82817 P
(43) Veröffentlichungstag der Anmeldung: 08.11.2000
(73) Patentinhaber: Mohr, Ulrich, 30559 Hannover (DE)
(72) Erfinder: MOHR, Ulrich, D-30559 Hannover (DE); AUFDERHEIDE, Michaela, D-30559 Hannover (DE)
(74) Vertreter: von Samson-Himmelstjerna, Friedrich
(86) Internationale Anmeldenummer: PCT/EP1999/000295
(87) Internationale Veröffentlichungsnummer: WO 1999/036505

(56) Entgegenhaltungen:
- GB-A- 2 055 397
- GB-A- 2 233 769
- GB-A- 2 314 343
- US-A- 4 370 418
- US-A- 4 812 407
- PATENT ABSTRACTS OF JAPAN vol. 096, no. 005, 31. Mai 1996 (1996-05-31) & JP 08 009958 A (SHIBAURA ENG WORKS CO LTD;OTHERS: 02), 16. Januar 1996 (1996-01-16)
- PATENT ABSTRACTS OF JAPAN vol. 006, no. 063 (C-099), 22. April 1982 (1982-04-22) & JP 57 005687 A (TORAY IND INC), 12. Januar 1982 (1982-01-12)
- PATENT ABSTRACTS OF JAPAN vol. 016, no. 431 (C-0983), 9. September 1992 (1992-09-09) & JP 04 148671 A (HITACHI LTD), 21. Mai 1992 (1992-05-21)
- PATENT ABSTRACTS OF JAPAN vol. 013, no. 516 (C-656), 17. November 1989 (1989-11-17) & JP 01 211486 A (SHIMADZU CORP), 24. August 1989 (1989-08-24)

## Beschreibung

Die Erfindung betrifft eine Kulturvorrichtung gemäß dem Oberbegriff des Anspruches 1 und ein Verfahren zur Kultivierung von Zellen oder Gewebekomponenten.

Derartige bekannte Kulturvorrichtungen dienen zur Kultivierung von isolierten Zellen oder Gewebekomponenten. Diese bekannten Kulturvorrichtungen sind auf den Einsatz bei Submerskultursystemen beschränkt, d.h. auf der Membran eines Zellkulturinserts kultivierte Zellen werden durch Überschichtung mit einer Kulturlösung mit den entsprechenden Nährstoffen versorgt.

Für manche experimentelle Studien, zum Beispiel Studien mit differenzierten Flimmer- oder Lungenepithelzellen, wäre es wünschenswert, wenn man zur Lösung spezieller Fragestellungen die Gewebewachstumsbedingungen variieren könnte. Mit herkömmlichen Kulturvorrichtungen läßt sich zum Beispiel eine Kultivierung von Zellen des Respirationstraktes an einer Luft/-Flüssigkeitsgrenzschicht (Air-Liquid) nicht durchführen, selbst wenn man poröse Membranen verwendet, die von verschiedenen Firmen als sogenannte Transwellsysteme angeboten werden.

Durch die vorliegende Erfindung soll eine Kulturvorrichtung gemäß dem Oberbegriff des Anspruches 1 so weitergebildet werden, daß sie eine Vielzahl unterschiedlicher Kultur- und Expositionsbedingungen ermöglicht.

Diese Aufgabe ist erfindungsgemäß gelöst durch ein Kulturvorrichtung mit den in Anspruch 1 angegebenen Merkmalen.

Bei der erfindungsgemäßen Kulturvorrichtung kann man die Höhe des Pegels des Kulturmediums im Inneren des Kulturgefäßes einstellen, insbesondere so, daß sie über der Oberseite der Zellkulturinsert-Membran liegt (Submersionskultur) oder gerade noch die Membran kontaktiert (oder etwas innerhalb derselben liegt), wodurch man eine basale, von unten erfolgende Nährstoffversorgung hat.

Das Umstellen von submerser Kultur auf basale Kultur kann mit der erfindungsgemäßen Kulturvorrichtung einfach durch Ansteuerung der Versorgungseinrichtung erfolgen, welche vorgegebene Mengen an Kulturmedium dem Kulturgefäß zuführt bzw. von diesem abführt, bis das Ausgangssignal des Pegelfühlers einem jeweils eingestellten Pegelsollwert entspricht.

Diese Einstellbarkeit des Pegels des Kulturmediums im Kulturbehälter läßt sich sehr präzise und einfach ohne Ablesen von Marken oder dergleichen leicht bewerkstelligen. Die zur Schaffung der unterschiedlichen Kulturbedingungen notwendigen apparativen Teile sind einfach und nicht teuer.

Vorteilhafte Weiterbildungen der Erfindung sind in den Unteransprüchen angegeben.

Mit der Weiterbildung der Erfindung gemäß Anspruch 2 wird erreicht, daß der Unterschied zwischen submerser und basaler Ernährung sehr scharf einstellbar ist. Die verschiedenen Bereiche einer vom Zellkulturinsert getragenen Zellkultur wachsen unter exakt den gleichen Bedingungen.

Bei einer Kulturvorrichtung gemäß Anspruch 3 kann man das in den einzelnen Kulturbehältern befindliche Kulturmedium während der Wachstumsphase analysieren und aus der Zusammensetzung des Kulturmediums Rückschlüsse auf das Wachsen der Zellkulturen über deren Stoffwechselprodukte schließen.

Bei einer Kulturvorrichtung gemäß Anspruch 4 kann man eine größere Anzahl von Zellkulturen unter exakt gleichen Bedingungen züchten. Auch der apparative Aufbau einer derartigen Kulturvorrichtung für eine große Anzahl von Zellkulturen ist einfach. Die Kulturvorrichtung benötigt auch nur wenig Platz.

Mit der Weiterbildung der Erfindung gemäß Anspruch 5 wird erreicht, daß für die verschiedenen Kulturbehälter nur ein einziger Pegelfühler zur Pegelregelung benötigt wird.

Die Weiterbildung der Erfindung gemäß Anspruch 6 ermöglicht es, den Pegel innerhalb der Kulturbehälter auf einfache Weise von außen festzustellen. Die Behälter können somit eine glatte Oberfläche aufweisen und sind frei von Einbauten, welche das Reinigen beeinträchtigen würden. Auch können die Kulturbehälter so einfach bei hohen Temperaturen sterilisiert werden.

Die Weiterbildung der Erfindung gemäß Anspruch 7 ermöglicht es, auch manuell den Sollpegel für das Kulturmedium einzustellen.

Gemäß Anspruch 8 ist es möglich, die Einstellung des Sollpegels auf einfache Weise elektrisch vorzunehmen. Der Pegelfühler selbst mißt über einen größeren Höhenbereich des Kulturbehälters, und die Pegelvorgabe erfolgt durch Einstellung des Pegelsollwertsignales.

Verwendet man zur Pegeldetektion gemäß Anspruch 9 Lichtschranken, insbesondere eine Gabellichtschranke, so kann man Komponenten verwenden, die zu günstigen Preisen als Standardbauteile vorliegen.

Die Weiterbildung der Erfindung gemäß Anspruch 10 ist im Hinblick auf eine nochmalige Vergrößerung der Anzahl gemeinsam durchführbarer Zellkulturen von Vorteil. Der apparative Aufwand für eine derartige Multi-Kulturvorrichtung ist klein. Diese besteht aus Standardbauteilen (Modulen), die in Großserie angefertigt werden können.

Die Weiterbildung der Erfindung gemäß Anspruch 11 ermöglicht, falls gewünscht, auch ein rückstandsfreies Abführen des Kulturmediums gleichermaßen von allen Modulen der Kulturvorrichtung.

Mit einer Kulturvorrichtung gemäß Anspruch 12 kann man in den verschiedenen Modulen unterschiedliche Zellkulturen züchten, hat aber trotzdem die Möglichkeit, den Fortschritt des Wachstums über die Stoffwechselprodukte für die verschiedenen Kulturen (die verschiedenen Module) getrennt zu untersuchen.

Die Weiterbildung der Erfindung gemäß Anspruch 13 ist im Hinblick auf gleiche Temperierung der verschiedenen Kulturen von Vorteil.

Mit der Weiterbildung der Erfindung gemäß Anspruch 14 wird ohne Verwendung von Rührern gewährleistet, daß sich innerhalb der einzelnen Temperierbehälter keine Temperaturgradienten ausbilden.

Bei einer Kulturvorrichtung gemäß Anspruch 15 ist die Verbindung zwischen den einzelnen Modulen der Kulturvorrichtung besonders einfach. Eine derartige Modulanordnung kann auch auf einfache Weise erweitert werden.

Bei einer Kulturvorrichtung gemäß Anspruch 16 ist der Innenraum der Kulturbehälter von der Umgebungsatmosphäre getrennt.

Bei einer solchen Kulturvorrichtung kann man dann gemäß Anspruch 17 den Innenraum des Außengehäuses auch mit einem anderen gasförmigen Medium füllen und dessen Einfluß auf das Wachstum der Kulturen untersuchen.

Die Weiterbildung der Erfindung gemäß Anspruch 18 ist in Hinblick auf das Schaffen einwandfreier keimfreier Ausgangsbedingungen für die Zellkulturen von Vorteil. Verwendet man dabei als sterilisierbares Material Glas, so hat man auch einen guten Blickkontakt zu den Zellkulturen Auch im Hinblick auf ein unerwünschtes Inlösunggehen des Materials der Kulturbehälter ist Glas als Material vorteilhaft.

Bei einer Kulturvorrichtung gemäß Anspruch 19 erfolgt sowohl das Zuführen als auch das Abführen von Kulturmedium zwangsweise durch eine bidirektional arbeitende Pumpenanordnung. Diese ist im Hinblick auf ein rasches Erreichen der Sollpegelwerte, also das Vermeiden von Regelschwingungen, von Vorteil.

Bei einer Kulturvorrichtung gemäß Anspruch 20 kann man die Kulturen gezielt Reizen aussetzen, aus denen sich eine Änderung der Wachstumsbedingungen ergibt. Außerdem kann man so die Kulturen gezielt Stoffen aussetzen, die sich nicht in der flüssigen Phase der Nährmedien befinden, wie gasförmigen Stoffen oder nicht in der flüssigen Phase dispergierbaren festen Stoffen.

Bei einer Kulturvorrichtung gemäß Anspruch 21 wechseln sich submerse Ernährung und basale Ernährung periodisch ab.

Mit einer Weiterbildung der vorliegenden Erfindung wird die Kulturvorrichtung über ein Programm gesteuert. Dieses Programm ist insbesondere unter Betriebssystemen MS-DOS lauffähig.

Das Programm ist dazu geeignet, alle Funktionen des Pumpsystems zu steuern. Typischerweise ist das Pumpsystem über eine serielle Schnittstelle an den Computer angeschlossen. Darüber hinaus detektiert und verarbeitet das Programm das Ausgangssignal der Photozelle. In einer besonders bevorzugten Ausführungsform wird der Versuchsablauf protokolliert.

Nachstehend wird die Erfindung anhand von Ausführungsbeispielen unter Bezugnahme auf die Zeichnung näher erläutert. In dieser zeigen:
- Fig. 1:: eine schematische Ansicht einer einfachen, nur einen Kulturbehälter umfassenden erfindungsgemäßen Kulturvorrichtung mit steuerbarer Pegelhöhe des Kulturmediums;
- Fig. 2:: eine ähnliche Ansicht wie Fig. 1, in welcher eine abgewandelte Kulturvorrichtung wiedergegeben ist;
- Fig. 3:: eine Aufsicht auf eine weiter abgewandelte Kulturvorrichtung, welche eine Mehrzahl parallel nebeneinander gestellter Kulturmodule aufweist, die ihrerseits jeweils eine Mehrzahl von Kulturbehältern aufweisen;
- Fig. 4:: einen Längsschnitt durch einen der Kulturmodule der Kulturvorrichtung nach Fig. 3;
- Fig. 5:: einen abgewinkelten transversalen Schnitt durch den Kulturmodul nach Fig. 4 längs der dortigen Schnittlinie V-V;
- Fig. 6:: einen longitudinalen Schnitt durch die Kulturvorrichtung nach Fig. 3;
- Fig. 7:: einen transversalen Schnitt durch die Kulturvorrichtung nach Fig. 3 längs der dortigen Schnittlinie IV-IV; und
- Fig. 8:: ein Blockschaltbild einer Pegelhöhensteuerung, die in Verbindung mit den Kulturvorrichtungen nach den Fig. 1 bis 6 Verwendung findet.

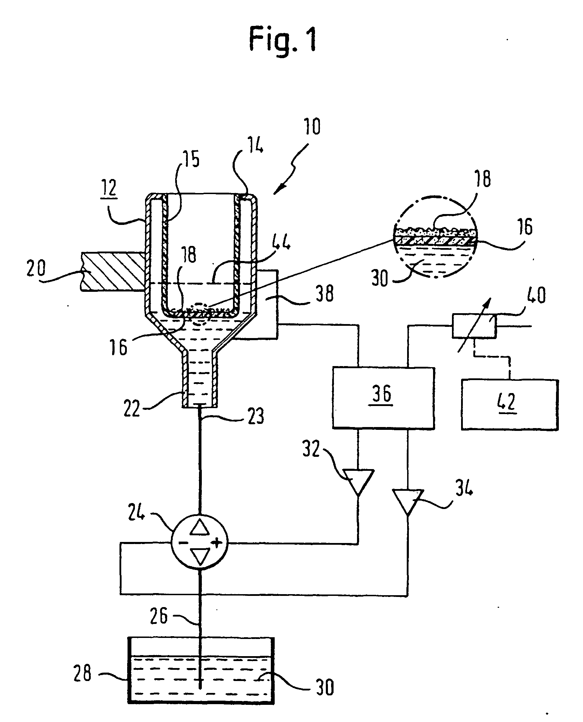

In Fig. 1 ist mit 10 insgesamt eine Kultureinheit bezeichnet. Die Kultureinheit 10 umfaßt einen Kulturbehälter 12, welcher grob gesprochen die Form einer auf den Kopf gestellten Flasche aufweist, wobei im Flaschenboden eine kreisförmige Öffnung 14 vorgesehen ist.

Im Inneren des Kulturbehälters 12 ist ein Zellkulturinsert 15 angebracht, das aus einem porösen Kunststoffmaterial hergestellt ist, zum Beispiel aus Polyethylenterephthalat. Das Zellkulturinsert 15 stellt eine flüssigkeitsdurchlässige Tragstruktur für eine Membran 16 dar, die je nach Erfordernis der zu kultivierenden Zellen aus unterschiedlichen Kunststoffmaterialien hergestellt sein kann, z.B. ebenfalls Polyethylenterephthalat. Die Membran 16 trägt, wie in der Ausschnittsvergrößerung von Fig. 1 dargestellt, eine Zellkultur 18.

Der Kulturbehälter 12 ist von einer Halteeinrichtung 20 getragen, die in Fig. 1 nur schematisch angedeutet ist.

Der unten liegende Anschluß 22 des Kulturbehälters 12 steht über einen Schlauch 23 mit dem einen Arbeitsanschluß einer bidirektionalen Pumpe 24 in Verbindung, welche in der Praxis eine Peristaltikpumpe sein kann. Der andere Anschluß der Pumpe 24 steht über einen Schlauch 26 mit dem Inneren eines Vorratsbehälters 28 in Verbindung, der eine Kulturflüssigkeit 30 enthält. Diese enthält diejenigen Nährstoffe, die der Zellkultur 18 für das Wachsen zur Verfügung gestellt werden sollen.

Die beiden Steuereinklemmen der Pumpe 24 sind über Leistungsverstärker 32, 34 mit den Ausgängen eines Betriebskreises 36 verbunden. Der Betriebskreis 36 erzeugt abhängig vom Ausgangssignal eines kontinuierlich arbeitenden Pegelfühlers 38, der als an der Außenfläche des Kulturbehälters 12 angebrachter Fühler dargestellt ist (aber gegebenenfalls auch an der Innenfläche des Kulturbehälters 12 angebracht sein kann) und in der Praxis ein optischer Fühler sein kann, und in Abhängigkeit von einem Sollwertgeber 40, der als einstellbarer Widerstand wiedergegeben ist, am einen, am anderen oder keinem seiner Ausgänge ein Signal, um die Pumpe 24 zu veranlassen, dem Inneren des Kulturbehälters 12 aus dem Vorratsbehälter 28 zusätzliche Kulturflüssigkeit zuzuführen oder Kulturflüssigkeit aus dem Inneren des Kulturbehälters 12 in den Vorratsbehälter 28 zurückzuleiten.

Der Sollwertgeber 40 kann von einer Programmsteuerung 42 her eingestellt werden, wie in Fig. 1 durch eine gestrichelte Linie angedeutet.

Die Programmsteuerung 42 schaltet beim hier betrachteten Ausführungsbeispiel den Sollwertgeber 40 zwischen zwei Stellungen um. Die eine Stellung des Sollwertgebers 40 entspricht einer Pegelhöhe der Kulturflüssigkeit 30 im Inneren des Kulturbehälters 12, wie er in der Zeichnung angegeben ist: die Kulturflüssigkeit 30 berührt gerade die Unterseite des Zellkuturinserts 15. Unter diesen Bedingungen wird der Zellkultur 18 nur von unten Kulturflüssigkeit zugeführt (basale Ernährung).

Der zweite von der Programmsteuerung 42 eingestellte Pegelsollwert entspricht einer Pegellage, wie sie in Fig. 1 durch die gestrichelte Linie 44 angedeutet ist. In dieser Stellung liegt der Flüssigkeitspegel über den Spitzen der Zellkultur 18; diese wird somit submers ernährt.

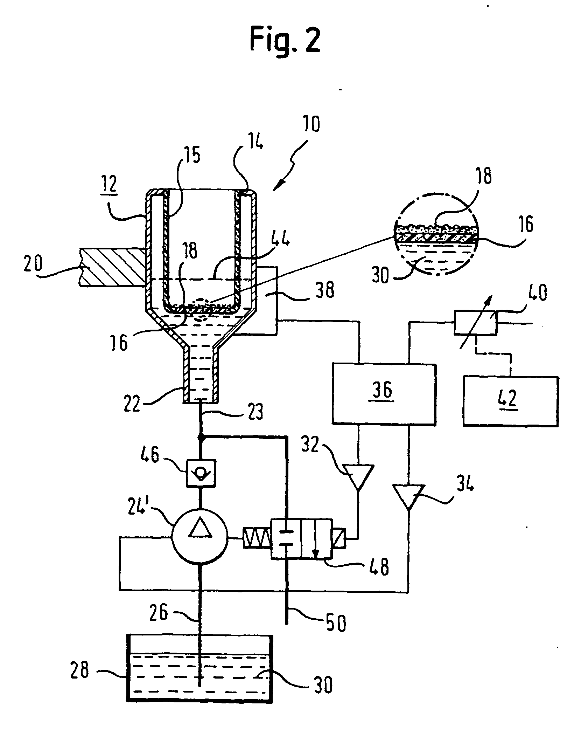

Das in Fig. 2 gezeigte Ausführungsbeispiel entspricht weitgehend demjenigen nach Fig. 1, entsprechende Komponenten sind wieder mit denselben Bezugszahlen versehen und werden nachstehend nicht nochmals detailliert erläutert.

Anstelle der bidirektional arbeitenden Pumpe 24 ist eine unidirektional arbeitende Förderpumpe 24' vorgesehen, die über ein Rückschlagventil 46 mit dem Anschluß 22 des Kulturbehälters 12 verbunden ist. Letztere ist ferner über ein 2/2-Magnetventil 48 mit einer Auslaßleitung 50 verbunden. Das Magnetventil 48 ist federnd in die Schließstellung vorgespannt und kann durch Aktivierung seines Magneten in die Durchlaßstellung gebracht werden. Der Magnet des Magnetventiles 48 und die Pumpe 24' sind wieder über die Leistungsverstärker 32, 34 mit dem Betriebskreis 36 verbunden, wie oben beschrieben, welcher einen Regelkreis bildet.

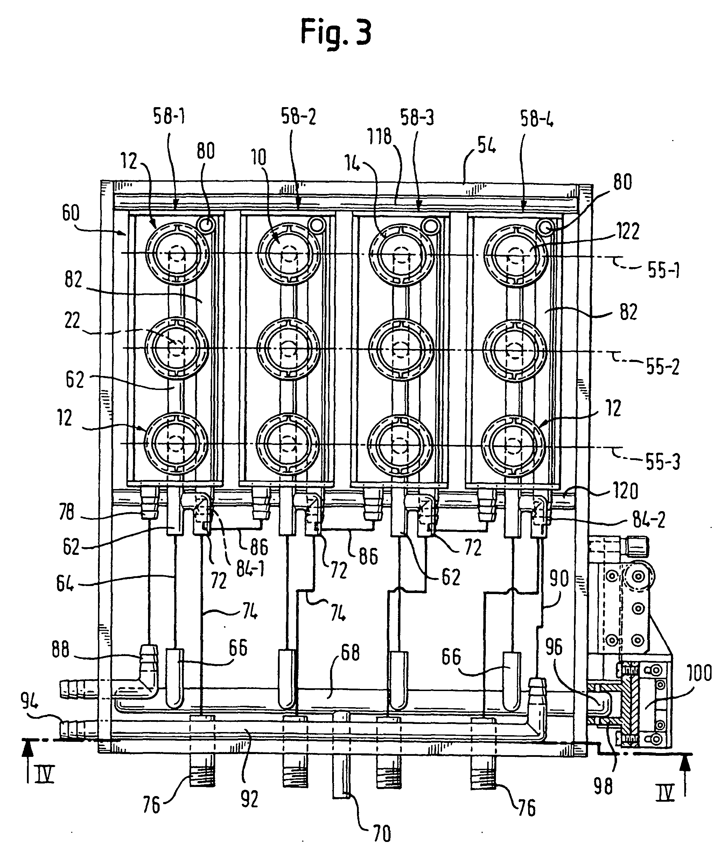

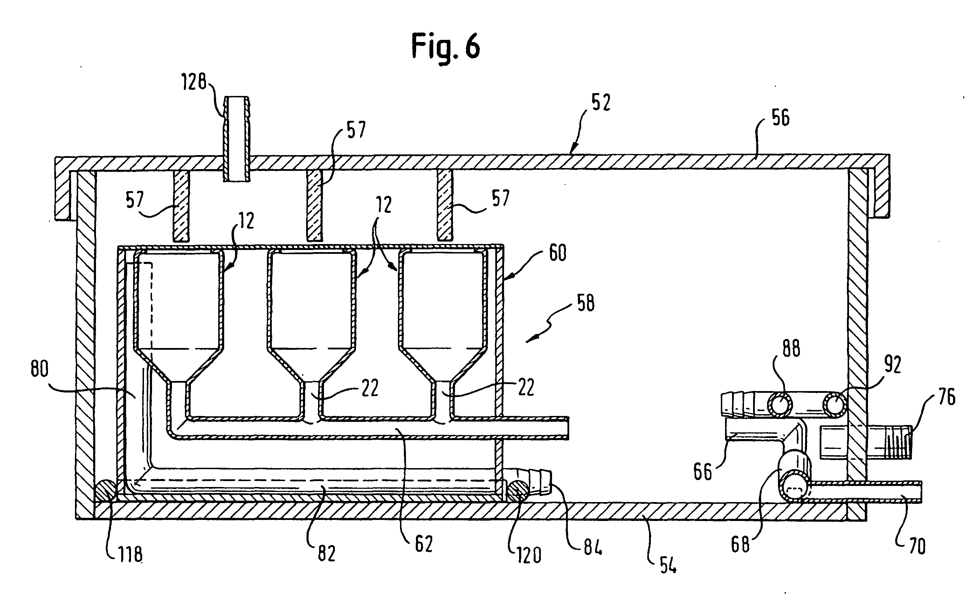

Die Figuren 3 bis 7 zeigen eine Kulturvorrichtung, mit welcher eine größere Anzahl von Zellkulturen gleichzeitig durchgeführt werden kann.

Die in den Figuren 3 bis 7 gezeigte Kulturvorrichtung hat ein dicht verschließbares Außengehäuse 52, welches aus einem unteren kastenförmigen, aus Platten zusammengesetzten Gehäuseteil 54 und einem dicht auf diesem aufsetzbaren Deckel 56 besteht, aus welchem sich senkrecht nach unten entlang der Lineien 55-1, 55-2 und 55-3 drei Glasstege 57 bis zum oberen Rand der Kulturbehälter 12 erstrecken, um darin angebrachte Zellkulturinserte 122 zu fixieren.

Im Inneren des Außengehäuses 52 sind vier Kulturmodule 58-1, 58-2, 58-3 und 58-4 angeordnet. Soweit nachstehend keine Unterscheidung der Kulturenmodule notwendig ist, wird auf diese mit dem Bezugszeichen 58 Bezug genommen.

Jeder der Kulturmodule 58 umfaßt ein insgesamt mit 60 bezeichnetes Temperiergehäuse, welches dicht ist und aus verschiedenen Platten zusammengefügt ist. Im Inneren eines Kulturgehäuses 60 sind jeweils drei Kultureinheiten 10 angeordnet, wie aus der Zeichnung ersichtlich. Die Anschlüsse 22 der verschiedenen Kulturbehälter 12 dieser Kultureinheiten sind mit einer gemeinsamen Versorgungsleitung 60 verbunden.

Die Versorgungsleitungen 62 der verschiedenen Kulturmodule 58 sind jeweils über einen nur schematisch wiedergegebenen Schlauch 64 aus Silicon mit einem zugeordneten Auslaßstutzen 66 eines Kulturmediumverteilers 68 verbunden. Der Kulturmediumverteiler 68 steht mit einem Zuführstutzen 70 für Kulturmedium in Verbindung, der ähnlich wie beim Ausführungsbeispiel nach Fig. 1 oder 2 mit dem Auslaß einer Kulturmedium-Pumpe 24 bzw. 24' über einen nicht dargestellten Silicon-Schlauch verbunden ist.

Die Versorgungsleitungen 62 haben jeweils bei ihrem außerhalb des zugeordneten Temperiergehäuses 60 liegenden Anschlußende einer Abzweig 72, der über einen Silicon-Schlauch 74 mit einem zugeordneten Entnahmestutzen 76 des Außengehäuses 52 verbunden ist. Auf diese Weise läßt sich zu Testzwecken Kulturflüssigkeit 30 aus den verschiedenen Modulen 58 getrennt abziehen.

Die Kulturbehälter 12 eines Kulturmoduls 58 sind gleichermaßen von dem über einen Temperiermedium-Anschlußstutzen 78 zugeführten Temperiermedium (z.B. temperiertes Wasser) umströmt. Das Abführen des zugeführten Temperiermediums erfolgt durch einen Überlauf 80, der in der Nachbarschaft der dem Anschlußstutzen 78 diametral gegenüberliegenden Ecke des Temperiergehäuses 60 vorgesehen ist.

Der Überlauf 80 steht über ein winkelförmiges Rohrstück 82 mit einem Auslaßstutzen 84 des Temperiergehäuses 60 in Verbindung. Der Auslaßstutzen 84 eines Kulturmoduls 58 ist jeweils mit dem Anschlußstutzen 78 des parallel daneben stehenden nächsten Kulturmoduls 58 verbunden, z.B. durch einen Schlauch 86. Auf diese Weise sind die Temperiergehäuse 60 nacheinander vom gleichen Temperiermedium durchströmt.

Der erste Anschlußstutzen 72 steht mit einem Temperierflüssigkeits-Speisestutzen 88 in Verbindung, der vom Außengehäuse 52 getragen ist. Der Auslaßstutzen 84-1 eines Moduls steht über einen Schlauch 86 mit dem Anschlußstutzen des folgenden Moduls in Verbindung. Der letzte Auslaßstutzen 84-2 der Modulanordnung steht über einen Schlauch 90 und ein Rohrstück 92 mit einem Temperiermedium-Auslaßstutzen 94 in Verbindung.

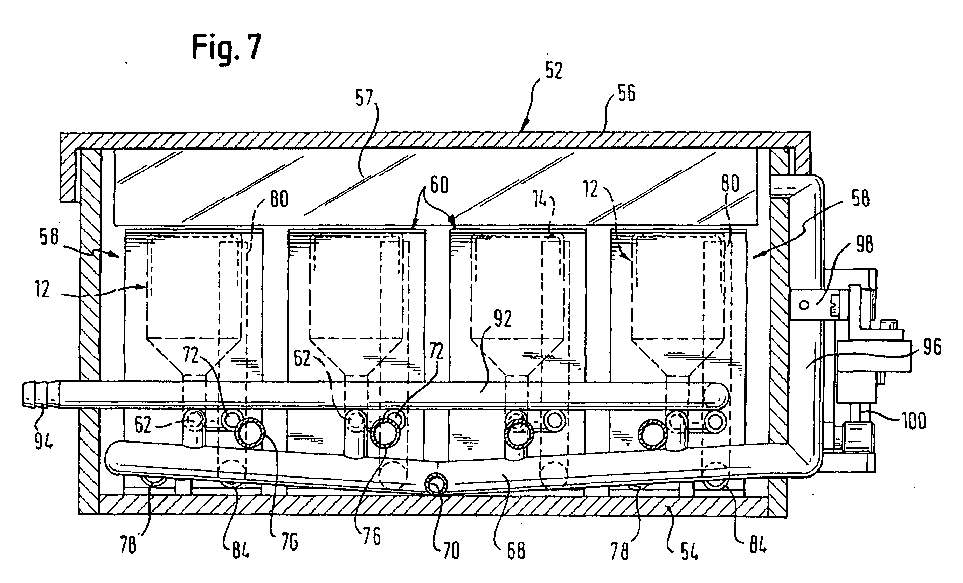

Wie aus der Zeichnung ersichtlich, steht mit dem Ende der Versorgungsleitung 62 ein Steigrohr 96 in Strömungsverbindung. Dieses steht senkrecht auf der Zeichenebene von Fig. 3, wie besonders gut aus Fig. 7 ersichtlich.

Aus Fig. 7 ist ferner gut ersichtlich, daß der Kulturmediumverteiler 68 in Frontansicht die Form eines weit geöffneten "V" aufweist, wobei der Zuführstutzen 70, der zugleich auch zum Abziehen überschüssiger Kulturflüssigkeit dient, bei der tiefsten Stelle des Kulturmediumverteilers 68 vorgesehen ist.

Die beiden Arme einer insgesamt mit 98 bezeichneten Gabellichtschranke übergreifen die Außenwand des Steigrohres 96. Die Gabellichtschranke 98 ist in vertikaler Richtung einstellbar von einem Träger 100 getragen, der sich parallel zu einer der Außenplatten des Außengehäuses 52 in vertikaler Richtung erstreckt.

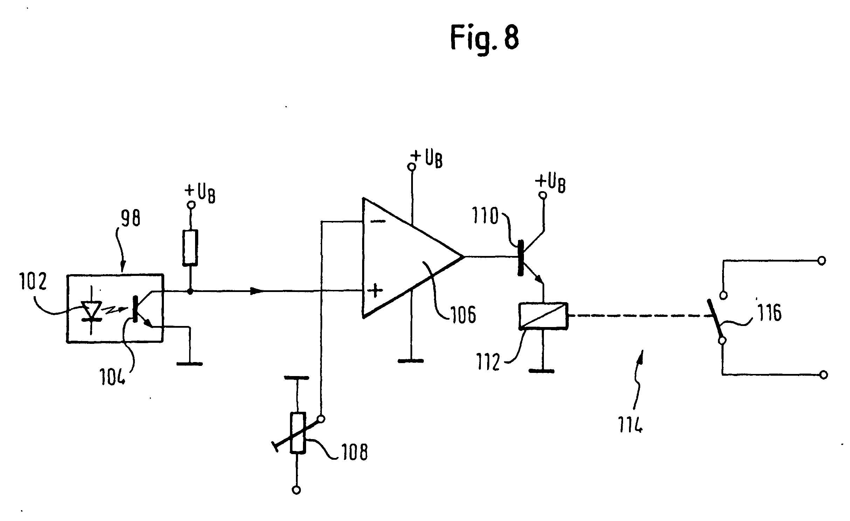

In den Armen der Gabellichtschranke 98 finden in üblicher Weise eine IR-Lichtquelle 102 (vgl. Fig. 8) in Form einer IR-Diode bzw. ein IR-Detektor 104 in Form eines IR-empfindlichen Phototransistors Verwendung. Liegt der Flüssigkeitspegel im Steigrohr 96, der dem Flüssigkeitspegel der exakt horizontal ausgefluchteten verschiedenen Kulturbehälter 12 entspricht, unterhalb der oben beschriebenen IR-Lichtschranke, erhält der Phototransistor 104 Strom und steuert durch. Ist die Flüssigkeitssäule im Steigrohr 96 bis in Höhe der Achse der durch die IR-Lichtquelle 102 und des IR-Detektors 104 gebildeten Lichtschranke angestiegen, verschwindet das Ausgangssignal des Phototransistors. Dies wird von einem Differenzverstärker 106 erkannt, der an seinem Seiteneingang das Ausgangssignal eines wieder als einstellbarer Widerstand gezeigten Sollwertgebers 108 erhält.

Durch das Ausgangssignal des Differenzverstärkers wird über einen Schalttransistor 110 die Relaisspule 112 eines insgesamt mit 114 bezeichneten Reed-Relais 114 aktiviert. Hierdurch wird der Reed-Kontakt 116 geschlossen, der ein potentialfreier Kontakt ist und zum Beispiel das Arbeiten der Pumpe 24 in Richtung eines Zuführens weiterer Kulturflüssigkeit 30 steuert.

Bei einer Einfachausführung der Kulturvorrichtung erfolgt das Umstellen von submerser Ernährung auf basale Ernährung zum Beispiel dadurch, daß man die Gabellichtschranke 98 entsprechend weit nach unten verschiebt, dann die überschüssige Kulturflüssigkeit durch manuelles Entleeren auf eine deutlich unter dem für basale Ernährung gewünschten Pegel liegenden Pegel absenkt und dann den Flüssigkeitspegel unter Steuerung durch das Ausgangssignal der Gabellichtschranke 98, die nun verstellt ist, auf das Niveau für basale Ernährung ansteigen läßt.

Für eine automatische Umschaltung zwischen diesen beiden Ernährungsvarianten wird vorzugsweise aber eine zweite Gabellichtschranke vorgesehen, die fest auf dem Niveau für basale Ernährung eingestellt wird. Für diese zweite Gabellichtschranke ist dann eine zweite Regelstrecke vorgesehen, die derjenigen nach Fig. 8 entspricht, mit dem Unterschied, daß der Reed-Kontakt 116 dann das Abführen von Kulturflüssigkeit steuert, also das Arbeiten einer bidirektionalen Pumpe im Sinne eines Abführens von Kulturflüssigkeit.

Die verschiedenen mechanischen Komponenten der oben beschriebenen Kulturvorrichtung sind, soweit sie starre Komponenten sind, vorzugsweise aus Glas ausgeführt. Diese Komponenten können somit bei Temperaturen von 120°C problemlos sterilisiert werden und können vor dem Durchführen von Versuchen in einen einwandfreien keimfreien Ausgangszustand vesetzt werden. Die verschiedenen oben beschriebenen Schlauchverbindungen bestehen vorzugsweise aus Silicon-Schlauchmaterial, welches ebenfalls bei 120°C problemlos sterilisierbar ist.

Wie oben dargestellt, wird zur Einregelung des Pegels der Kulturflüssigkeit in den verschiedenen Kulturbehältern 12 nur ein einziger Pegelfühler verwendet. Aus diesem Grunde ist es notwendig, die verschiedenen Kulturbehälter 12 alle exakt auszufluchten, derart, daß die Flächen, auf denen die Zellkulturen wachsen, in einer gemeinsamen horizontalen Ebene liegen. Für eine Zwangspositionierung der verschiedenen Kulturmodule und für ein Verrasten der Kulturmodule im Außengehäuse sind zwei Positionierleisten 118 und 120 vorgesehen, die mit den Vorderkanten bzw. Hinterkanten der unteren Platten der Temperiergehäuse 60 zusammenarbeiten.

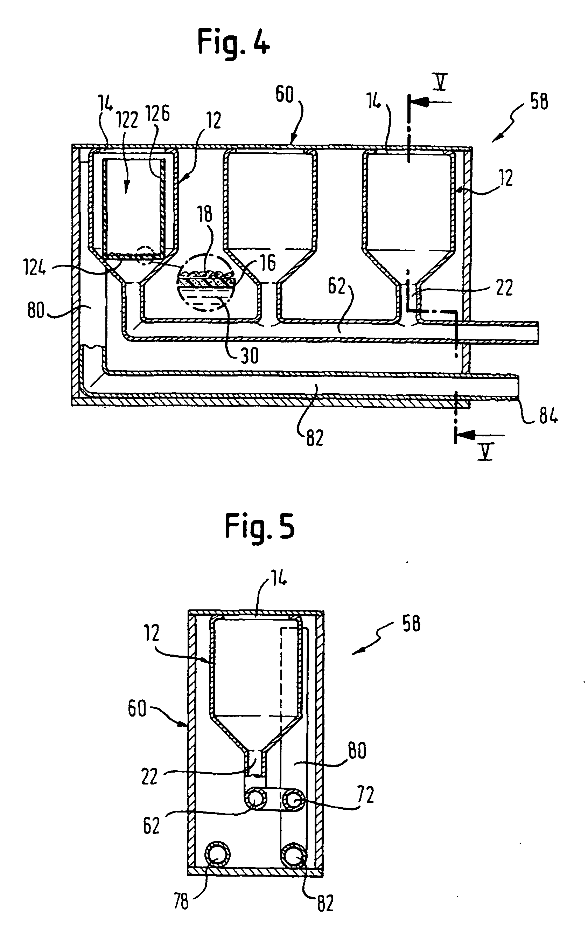

Bei den Prinzipdarstellungen in den Figuren 1 und 2 waren die Zellkulturinserte 15 schematisch als eingebaute Substrate dargestellt. In der Praxis handelt es sich bei den Zellkulturinserten 15 nicht um fest in die Kulturbehälter 12 eingebaute Komponenten, vielmehr um aus diesen entnehmbare Teile. Kommerzielle Zellkulturinserte haben, wie in Fig. 4 bei 122 für einen der Kulturbehälter 12 angedeutet, die Form eines zylindrischen Bechers mit einer ebenen Bodenwand 124 und einer zylindrischen Umfangswand 126. Durch Einsetzen der Zellkulturinserte 122 in die Kulturbehälter 12 und Fixierung mittels der Glasstege 57 ist gewährleistet, daß die verschiedenen Bodenwände 124 in einer gemeinsamen horizontalen Ebene liegen. Die Zellkulturinserte 122 sind insgesamt aus einem porösen Kunststoffmaterial hergestellt, wie auch die Zellkulturinserte 15 der Figuren 1 und 2.

Wie oben dargestellt, kann man bei der beschriebenen Kulturvorrichtung sehr einfach von submerser auf basale Ernährung der Zellkulturen umstellen. Durch Änderung der Temperatur der zugeführten Temperierflüssigkeit kann man eine weitere Variation der Wachstumsbedingungen vornehmen. Schließlich kann man dadurch, daß man dem Inneren des Außengehäuses 52 über eine Leitung 128 gasförmige Substanzen zuführt, die Zellkulturen vorgegebenen schädigenden oder therapeutischen Bedingungen aussetzen. Auf die gleiche Weise kann auch eine Exposition mit Aerosolen erfolgen.

Analog zur direkten Behandlung mit Gasen und/oder Aerosolen lassen sich auch partikuläre Wirkstoffe unmittelbar mit den Zellkulturen in Kontakt bringen. Hierzu werden die Partikel, deren Auswirkung untersucht werden soll, auf die Zellkulturen aufgestäubt. Stimmt man die Dichten der Partikel und der Kulturflüssigkeit so aufeinander ab, daß die Partikel leichter sind als die Kulturflüssigkeit, so werden die Partikel mit dem Anheben des Pegels der Kulturflüssigkeit von der Zellkultur abgehoben. Ist die Kulturflüssigkeit dagegen abgesenkt (basale Ernährung), stehen die Partikel in Kontakt mit der Zellkultur. Derartige geeignete Partikel sind sehr feine Stäube, z.B. Holzstaub oder auch hydrophobe Partikel, welche sich in Suspension nur unzureichend oder gar nicht absetzen.

Zur direkten Begasung der Zellen mit Stoffen wie z.B. Ozon oder zur Beaufschlagung der Zellen mit Partikeln und komplexen Stoffgemischen unmittelbar an der Luft/Flüssigkeitsgrenze unter Einhaltung der Pulskulturtechnik können zur weiteren Aufrüstung der Kultureinheit spezielle Aufsätze für die Kulturmodule eingesetzt werden.

Auch können Biosensoren zur kontinuierlichen oder diskontinuierlichen Erfassung spezieller Zellbestandteile (z.B. Calcium) und -produkte sowie Meßsonden und Meßvorrichtungen zur kontinuierlichen oder diskontinuierlichen Messung von gasförmigen (z.B. CO₂, NOₓ) oder partikulären Bestandteilen ("total particulate matter", TPM) luftgetragener Stoffe in der Expositionsatmosphäre der Zellen an die Kulturmodule adaptiert werden.

Diese verschiedenen Möglichkeiten der Zellkultur werden bei einfachem apparativen Aufbau der Kulturvorrichtung erhalten.

Die oben beschriebenen Kulturvorrichtungen eignen sich gut für den universellen Einsatz käuflicher Zellkulturinserte. Die modulare Bauweise der Vorrichtung erlaubt auch eine problemlose Adaptation der Kulturbehälter ("Wells") an spezifische Insertgrößen.

Die Durchführung der Versuche ist sehr flexibel, pro Modul können drei parallele Versuchsansätze gefahren werden. Unter Einsatz aller Module der Kulturvorrichtung nach den Figuren 3 bis 7 können vier mal drei jeweils parallele Versuchsansätze gefahren werden. Dies erlaubt es zum Beispiel, zelluläre Reaktionen in Abhängigkeit von Konzentration und/oder Zeit zu ermitteln.

Verschiedene spezielle zellbiologische Fragestellungen, die mit einer erfindungsgemäßen Kulturvorrichtung gelöst werden können, sind zum Beispiel:
1. Behandlung von Zellen mit z.B. Gasen, Aerosolen oder Stäuben unmittelbar an der Luft-/Flüssigkeitsgrenzschicht.
2. Zelluläre Interaktionen durch Cokultivierung verschiedener Zelltypen, z.B. von Epithelzellen und Fibroplasten.
3. Anheftungsverhalten von Zellen in einem System, das pulsierend mit Kulturflüssigkeit versorgt wird.
4. Differenzierung der Zellen von Kulturen bei basaler Kulturflüssigkeitsversorgung (Polarisierung).
5. Untersuchung zilientragender Zellen bei basaler und/oder intermittierender Versorgung mit Kulturflüssigkeit.
6. Kontinuierliche Analyse von Zellausscheidungsprodukten während der Versuche.
7. Intermittierende Zugabe von wachstumsstimulierenden und wachstumsinhibierenden Wirkstoffen ohne Unterbrechung der Kulturführung.
8. Intermittierende Zugabe von den unter 6. genannten Stoffen während der Exposition von Zellen mit Wirk- oder Schadstoffen.

In den Figuren 3 bis 7 wurde eine Kulturvorrichtung gezeigt, die eine eigene Temperiereinrichtung hat. Sie kann jedoch, ebenso wie Kulturvorrichtungen ohne Temperiereinrichtung, wie sie in den Figuren 1 und 2 gezeigt sind, zur Temperierung einfach in einen Temperierschrank gestellt werden.

Die Vorbereitung der Kulturvorrichtung für einen Versuch verläuft im einzelnen folgendermaßen:

Die Kulturvorrichtung wird in zusammengebautem Zustand, jedoch ohne Zellkulturinserte, Versorgungseinrichtung und Pegelfühler, bei 120°C im Autoklaven für 30 Minuten autoklaviert, ebenso sonstige benötigte Materialien, wie Pumpenschläuche, Pinzetten zum Einsetzen der Zellkulturinserte, Flaschen zur Aufnahme des Kulturmediums usw. Nach dem Autoklavieren wird der Pegelfühler in einer Reinraumwerkbank montiert. Danach erfolgt der Anschluß des Mediumsschlauches an die Pumpe und die Einstellung der Pumpengeschwindigkeit für die vorgesehenen Versuchsbedingungen. Vor dem Einsetzen der Zellkulturinserte, die einzeln verpackt und steril vom Hersteller geliefert werden, wird die Kulturvorrichtung einmal mit dem Zelltyp-spezifischen Kulturmedium gefüllt und wieder entleert (gespült), wonach das dabei verwendete Medium verworfen wird. Anschließend wird die gesamte Kulturvorrichtung bis unterhalb der Kulturgefäße mit dem Zelltyp-spezifischen Medium gefüllt, und die vorbereiteten Zellkulturinserte werden in der Reinraumwerkbank mittels einer Pinzette eingesetzt. Anschließend wird das gewählte Programm zur Pumpensteuerung gestartet, und die Kulturvorrichtung wird gegebenenfalls in den Temperierschrank überführt.

Das Programm kontrolliert einen Zyklus, der aus den Schritten (1) Vorpumpen und Kulturzeit und (2) Rückpumpen und Warten besteht.

Nach Initialisierung führt die Pumpe ihre Vorpump-Funktion eigenständig durch. Hierzu wird zuvor je nach Zelltyp und Kultivierungsart die adäquate Pumpgeschwindigkeit gewählt. Das Programm überwacht das Ausgangssignal der Photozelle und regelt die Beendigung der Pumpaktivität, sobald der gewünschte Pegelstand erreicht ist. Typischerweise erfolgt die Beendigung der Pumpaktivität erst nach zwei- oder mehrmaliger, vom Programm überwachter Signaldetektion. Dies erlaubt die Vermeidung der Detektion von Fehlsignalen, die bspw. durch Blasenbildung hervorgerufen werden können und bei einmaliger Signaldetektion zu einer vorzeitigen und unerwünschten Beendigung der Pumpaktivität führen würden. Die nachfolgende Kulturzeit, ist bevorzugt wähl- und einstellbar.

Nach Ablauf der Kulturzeit wird der Abpumpvorgang des Mediums initialisiert, wobei wiederum die Pumpgeschwindigkeit wähl- und einstellbar ist. Aus der gewählten Abpumpgeschwindigkeit und dem Volumen des Mediums ergibt sich die für den Abpumpvorgang erforderliche Wartezeit.

Der vorgenannte, vom Programm gesteuerte Zyklus kann einmal, zweimal oder mehrmals durchlaufen werden.

Die Zellkulturinserte können jederzeit in der Reinraumwerkbank entnommen werden. Dazu wird das Computerprogramm angehalten. Falls nur einige Inserte entnommen werden, kann das Programm wieder gestartet werden.

Zur Reinigung wird die geamte Kulturvorrichtung mehrmals mit Aqua dest. gespült. Die Glasteile lassen sich problemlos auch mit Alkohol oder anderen Reinigungsmitteln säubern.

Obenstehend wurde die Erfindung anhand spezieller Pegelfühler beschrieben. Es versteht sich, daß auch andere Pegelfühler geeignet sind, die die Lage des momentanen Flüssigkeitspegels im Kulturbehälter 12 erkennen lassen und ein entsprechendes Ausgangssignal bereitstellen. Hierzu gehören schimmergesteuerte Fühler, nach dem dielektrischen Prinzip arbeitende Fühler, eine Mehrzahl in vertikaler Richtung beabstandeter Elektroden aufweisende Fühler und Infrarot- und Ultraschall-Fühler.

## Patentansprüche

1. Kulturvorrichtung, mit mindestens einem Kulturbehälter (12), welcher mindestens einen Anschluß (22) zum Zuführen und Abführen von Kulturmedium (30) aufweist, mit jeweils einem Zellkulturinsert (15; 122) für jeden der Kulturbehälter (12), das im Inneren desselben gehalten ist, und mit einer Versorgungseinrichtung (24 bis 28; 24', 48, 50) zum Zuführen von Kulturmedium (30) zu den Kulturbehältern (12) bzw. zum Abführen von Kulturmedium von diesen, **dadurch gekennzeichnet, daß** den Kulturbehältern (12) ein oder zwei auf den Pegel des Kulturmediums (30) ansprechende (r) Pegelfühler (38; 98) zugeordnet ist/sind und daß die Versorgungseinrichtung (24 bis 28; 24', 28, 48, 50) in Abhängigkeit vom Ausgangssignal eines Pegelfühlers oder von zwei Pegelfühlern (38; 98) so gesteuert wird, daß sowohl eine submerse als auch eine basale Kultivierung von Zellen möglich ist.

2. Kulturvorrichtung nach Anspruch 1, **dadurch gekennzeichnet, daß** die Zellkulturinserte (15; 122) jeweils eine horizontale Kulturfläche vorgeben.

3. Kulturvorrichtung nach Anspruch 1 oder 2, **gekennzeichnet durch** den einzelnen Kulturbehältern (12) oder Gruppen von Kulturbehältern (12) zugeordnete Entnahmeleitungen (74, 76).

4. Kulturvorrichtung nach einem der Ansprüche 1 bis 3, **dadurch gekennzeichnet, daß** eine Mehrzahl von Kulturbehältern (12) an eine gemeinsame Versorgungsleitung (62) derart angeschlossen sind, daß die Kulturflächen der Zellkulturinserte (15; 122) in einer gemeinsamen horizontalen Ebene liegen.

5. Kulturvorrichtung nach . Anspruch 4, **dadurch gekennzeichnet, daß** die Versorgungsleitungen (62) mit einem Steigrohr (96) kommunizieren, bei welchem der Pegelfühler (38; 98) vorgesehen ist.

6. Kulturvorrichtung nach einem der Ansprüche 1 bis 5, **dadurch gekennzeichnet, daß** ein oder beide Pegelfühler (38; 98) eine bzw. jeweils eine Gabellichtschranke umfassen.

7. Kulturvorrichtung nach Anspruch 5 oder 6, **dadurch gekennzeichnet, daß** der oder einer der beiden Pegelfühler (38; 98) auf dem Steigrohr (96) einstellbar ist.

8. Kulturvorrichtung nach einem der Ansprüche 1 bis 7, **dadurch gekennzeichnet, daß** ein oder beide Pegelfühler (38; 98) ein bzw. jeweils ein kontinuierlich den Pegel messender Fühler ist/sind.

9. Kulturvorrichtung nach einem der Ansprüche 1 bis 7, **dadurch gekennzeichnet, daß** ein oder beide Pegelfühler (38; 98) ein bzw. jeweils ein bei einem vorgegebenen Pegel ansprechender Pegelschalter ist.

10. Kulturvorrichtung nach einem der Ansprüche 5 bis 9, **dadurch gekennzeichnet, daß** sie mehrere Module (58) aufweist, die ihrerseits jeweils eine Mehrzahl von Kulturbehältern (12) umfassen, welche parallel ausgerichtet in einem Außengehäuse (52) angeordnet sind, wobei ein Versorgungsverteiler (68) vorgesehen ist, der mit Versorgungsleitungen (62) der einzelnen Module (58) verbunden ist.

11. Kulturvorrichtung nach Anspruch 10, **dadurch gekennzeichnet, daß** ein mit der Versorgungseinrichtung (24 bis 28; 24', 28, 48, 50) zu verbindender einziger Anschluß (70) des Versorgungsverteilers (68) an einer tiefsten Stelle desselben liegt.

12. Kulturvorrichtung nach Anspruch 10, **dadurch gekennzeichnet, daß** das Außengehäuse (52) getrennte Anschlüsse (76) für Entnahmeleitungen (74) aufweist, die jeweils zu einem der Kulturmodule (58) führen.

13. Kulturvorrichtung nach Anspruch 12, **dadurch gekennzeichnet, daß** die Kulturbehälter (12) in jeweils mindestens einen Kulturbehälter (12) umfassenden Modulen (58) angeordnet sind, die jeweils ein die Kulturbehälteranordnung umgebendes Temperiergehäuse (60) aufweisen, welches einen Temperiermedium-Einlaß (78) und einen Temperiermedium-Auslaß (84) aufweist.

14. Kulturvorrichtung nach Anspruch 13, **dadurch gekennzeichnet, daß** der Temperiermedium-Auslaß (84) mit einem im oberen Bereich des Temperiergehäuses (60) liegenden Überlauf (80) in Verbindung steht, der vorzugsweise dem Temperiermedium-Einlaß (78) diametral gegenüberliegt.

15. Kulturvorrichtung nach Anspruch 14, **dadurch gekennzeichnet, daß** die Temperiergehäuse (60) der verschiedenen Module (58) bezüglich des Temperiermediums strömungsmäßig in Reihe geschaltet sind.

16. Kulturvorrichtung nach einem der Ansprüche 1 bis 15, **dadurch gekennzeichnet, daß** die Kulturbehälter (12) von einem dicht verschließbaren Außengehäuse (52) umgeben sind.

17. Kulturvorrichtung nach Anspruch 16, **dadurch gekennzeichnet, daß** das Außengehäuse (52) mit einem Anschluß (128) für ein gasförmiges Medium versehen ist.

18. Kulturvorrichtung nach einem der Ansprüche 1 bis 17, **dadurch gekennzeichnet, daß** sie abgesehen von dem Pegelfühler und Teilen der Versorgungseinrichtung im wesentlichen aus einem sterilisierbaren Material, insbesondere Glas und Siliconmaterial, besteht.

19. Kulturvorrichtung nach einem der Ansprüche 1 bis 18, **dadurch gekennzeichnet, daß** die Versorgungseinrichtung (24 bis 28) eine bidirektional arbeitende Pumpenanordnung (24), insbesondere eine Peristaltikpumpe aufweist.

20. Kulturvorrichtung nach einem der Ansprüche 1 bis 19, **gekennzeichnet durch** eine Programmsteuerung 42, welche einen Sollpegelgeber 40 zeitabhängig steuert.

21. Kulturvorrichtung nach Anspruch 20, **dadurch gekennzeichnet, daß** die Programmsteuerung 42 den Sollpegelwert periodisch zwischen zwei Werten umschaltet, von denen der eine einem eine vorgegebene Strecke über der Oberseite der Kulturfläche liegenden Flüssigkeitspegel entspricht und der andere einen eine vorgegebene Strecke unter der Oberseite der Kulturfläche liegenden Pegel vorgibt.

22. Verfahren zu Kultivierung von Zellen oder Gewebekomponenten unter Verwendung einer Kulturvorrichtung nach einem der Ansprüche 1 bis 21, **dadurch gekennzeichnet, daß** der Flüssigkeitspegel einer Nährstofflösung für die Zellen oder Gewebeteile abwechselnd eine vorgegebene Strecke über der Oberseite einer Kultur der Zellen oder Gewebeteilen (submerse Kultivierung) und eine vorgegebene Strecke unter der Oberseite der Kultur (basale Kultivierung) liegt.

23. Verfahren nach Anspruch 22, **dadurch gekennzeichnet, daß** in einem Zeitraum, in dem der Flüssigkeitspegel unter der Oberseite der Kultur liegt, die Kultur festen, gasförmigen oder Aerosol-förmigen Substanzen ausgesetzt wird.

24. Verfahren nach Anspruch 22 oder 23, **dadurch gekennzeichnet, daß** die Lage des Flüssigkeitspegels mit Hilfe eines Pegelfühlers und einer **dadurch** gesteuerten Pumpeneinrichtung vorgenommen wird.

25. Verfahren nach einem der Ansprüche 22 bis 24, **dadurch gekennzeichnet, daß** eine zeitliche Einstellung der Lage des Flüssigkeitspegels mit Hilfe einer Programmsteuerung vorgenommen wird.

## Claims

1. Culture device with at least one culture container (12) which has at least one connection (22) for supplying and discharging culture medium (30), with in each case a cell culture insert (15; 122) for each of the culture containers (12), said culture insert being held inside the latter, and with a supply device (24 to 28; 24', 48, 50) for supplying culture medium (30) to the culture containers (12) and for discharging culture medium from the latter, **characterized in that** the culture containers (12) are assigned one or two level sensors (38; 98) which respond to the level of the culture medium (30), and **in that** the supply device (24 to 28; 24', 28, 48, 50) is controlled as a function of the output signal of a level sensor or of two level sensors (38; 98) in such a way that both submersed and basal cultivation of cells is possible.

2. Culture device according to Claim 1, **characterized in that** the cell culture inserts (15; 122) in each case have a horizontal culture surface.

3. Culture device according to Claim 1 or 2, **characterized by** take-off lines (74, 76) assigned to the individual culture containers (12) or groups of culture containers (12).

4. Culture device according to any of Claims 1 to 3, **characterized in that** a plurality of culture containers (12) are connected to a common supply line (62) in such a way that the culture surfaces of the cell culture inserts (15; 122) lie in a common horizontal plane.

5. Culture device according to Claim 4, **characterized in that** the supply lines (62) communicate with an ascending pipe (96) in which the level sensor (38; 98) is provided.

6. Culture device according to any of Claims 1 to 5, **characterized in that** one or both level sensors (38; 98) comprises or in each case comprise a forked light barrier.

7. Culture device according to Claim 5 or 6, **characterized in that** the level sensor or one of the two level sensors (38; 98) can be placed on the ascending pipe (96).

8. Culture device according to any of Claims 1 to 7, **characterized in that** one or both level sensors (38; 98) is or are respectively a sensor which continuously measures the level.

9. Culture device according to any of Claims 1 to 7, **characterized in that** one or both level sensors (38; 98) is or are respectively a level sensor which responds at a predefined level.

10. Culture device according to any of Claims 5 to 9, **characterized in that** it has a number of modules (58) which in turn respectively comprise a plurality of culture containers (12) which are arranged in parallel alignment in an outer housing (52), wherein a supply distributor (68) is provided which is connected to supply lines (62) of the individual modules (58).

11. Culture device according to Claim 10, **characterized in that** a single connection (70) of the supply distributor (68) which is to be connected to the supply device (24 to 28; 24', 28, 48, 50) lies at a lowest point of said supply distributor.

12. Culture device according to Claim 10, **characterized in that** the outer housing (52) has separate connections (76) for take-off lines (74) which in each case lead to one of the culture modules (58).

13. Culture device according to Claim 12, **characterized in that** the culture containers (12) are arranged in modules (58) which in each case comprise at least one culture container (12), said modules in each case having a temperature-controlled housing (60) which surrounds the culture container arrangement and has a temperature control medium inlet (78) and a temperature control medium outlet (84).

14. Culture device according to Claim 13, **characterized in that** the temperature control medium outlet (84) is connected to an overflow (80) located in the upper region of the temperature-controlled housing (60), said overflow preferably being located diametrically opposite the temperature control medium inlet (78).

15. Culture device according to Claim 14, **characterized in that** the temperature-controlled housings (60) of the various modules (58) are connected in series in the direction of flow of the temperature control medium.

16. Culture device according to any of Claims 1 to 15, **characterized in that** the culture containers (12) are surrounded by an outer housing (52) which can be tightly sealed.

17. Culture device according to Claim 16, **characterized in that** the outer housing (52) is provided with a connection (128) for a gaseous medium.

18. Culture device according to any of Claims 1 to 17, **characterized in that**, apart from the level sensors and parts of the supply device, it is made essentially of a sterilizable material, in particular glass and silicone material.

19. Culture device according to any of Claims 1 to 18, **characterized in that** the supply device (24 to 28) has a pump arrangement (24) which operates in a bidirectional manner, in particular a peristaltic pump.

20. Culture device according to any of Claims 1 to 19, **characterized by** a program controller (42) which controls a desired level generator (40) in a time-controlled manner.

21. Culture device according to Claim 20, **characterized in that** the program controller (42) periodically switches the desired level generator between two values, of which one corresponds to a fluid level lying a predefined distance above the top of the culture surface and the other prescribes a level lying a predefined distance below the top of the culture surface.

22. Method for cultivating cells or tissue components using a culture device according to any of Claims 1 to 21, **characterized in that** the fluid level of a nutrient solution for the cells or tissue parts alternately lies a predefined distance above the top of a culture of the cells or tissue parts (submersed cultivation) and a predefined distance below the top of the culture (basal cultivation).

23. Method according to Claim 22, **characterized in that**, in a period in which the fluid level lies below the top of the culture, the culture is exposed to solid, gaseous or aerosol-type substances.

24. Method according to Claim 22 or 23, **characterized in that** the position of the fluid level is set by means of a level sensor and a pump device controlled thereby.

25. Method according to any of Claims 22 to 24, **characterized in that** temporal adjustment of the position of the fluid level is carried out by means of a program controller.

## Revendications

1. Dispositif de culture avec au moins un récipient de culture (12), qui présente au moins une jonction (22) pour l'apport et l'évacuation d'un milieu de culture (30), avec chaque fois un insert de culture cellulaire (15 ; 122) pour chacun des récipients de culture (12), qui est maintenu à l'intérieur de celui-ci, et avec un dispositif d'alimentation (24 à 28 ; 24', 48, 50) pour l'apport du milieu de culture (30) aux récipients de culture (12) ou pour l'évacuation du milieu de culture de ceux-ci, **caractérisé en ce que** les récipients de culture (12) présentent un ou deux détecteurs de niveau (38 ; 98) correspondant au niveau du milieu de culture (30) et **en ce que** le dispositif d'alimentation (24 à 28 ; 24', 48, 50) est réglé en fonction du signal d'entrée d'un détecteur de niveau ou de deux détecteurs de niveau (38 ; 98), de sorte que non seulement une culture immergée, mais également une culture basale de cellules sont possibles.

2. Dispositif de culture selon la revendication 1, **caractérisé en ce que** l'insert de culture (15; 122) prévoit chaque fois, une surface horizontale de culture.

3. Dispositif de culture selon la revendication 1 ou 2, **caractérisé par** des conduites de prélèvement (74, 76) rattachées aux récipients individuels de culture (12) ou aux groupes de récipients de culture (12).

4. Dispositif de culture selon l'une quelconque des revendications 1 à 3, **caractérisé en ce qu'**un certain nombre de récipients de culture (12) sont raccordés à une conduite d'alimentation commune (62), de sorte que les surfaces de culture des inserts de culture cellulaire (15; 122) se trouvent dans un plan horizontal commun.

5. Dispositif de culture selon la revendication 4, **caractérisé en ce que** les conduites d'alimentation (62) communiquent avec un tube ascendant pour lequel est prévu le détecteur de niveau (38 ; 98).

6. Dispositif de culture selon l'une quelconque des revendications 1 à 5, **caractérisé en ce qu'**un ou les deux détecteurs de niveau (38 ; 98) comprennent un ou chaque fois un barrage photoélectrique.

7. Dispositif de culture selon la revendication 5 ou 6, **caractérisé en ce que** le ou un des deux détecteurs de niveau (38 ; 98) peut être ajusté sur le tube ascendant (96).

8. Dispositif de culture selon l'une quelconque des revendications 1 à 7, **caractérisé en ce qu'**un ou les deux détecteurs de niveau (38 ; 98) sont un ou chaque fois un détecteur mesurant le niveau en continu.

9. Dispositif de culture selon l'une quelconque des revendications 1 à 7, **caractérisé en ce qu'**un ou les deux détecteurs de niveau (38 ; 98) sont un ou chaque fois un commutateur de niveau correspondant à un niveau donné.

10. Dispositif de culture selon l'une quelconque des revendications 5 à 9, **caractérisé en ce qu'**il présente plusieurs modules (58) qui comprennent pour leur part chaque fois un certain nombre de récipients de culture (12) qui sont disposés parallèlement dans un boîtier extérieur (52) où un distributeur d'alimentation (68) est prévu, lequel est relié aux conduites d'alimentation (62) de chaque module (58).

11. Dispositif de culture selon la revendication 10, **caractérisé en ce qu'**un seul raccord (70) du distributeur d'alimentation (68), à relier au dispositif d'alimentation (24 à 28 ; 24', 48, 50), se trouve sur un site plus profond de celui-ci.

12. Dispositif de culture selon la revendication 10, **caractérisé en ce que** le boîtier extérieur (52) présente des raccords séparés (76) pour les conduites de prélèvement (74), qui conduisent chaque fois à un module de culture (58).

13. Dispositif de culture selon la revendication 12, **caractérisé en ce que** les récipients de culture (12) sont disposés dans des modules comprenant chaque fois au moins un récipient de culture (12), lequel module présente chaque fois un boîtier de mise à température (60) entourant le dispositif de récipients de culture, qui présente une entrée de milieu de mise à température (78) et une sortie de milieu de mise à température (84).

14. Dispositif de culture selon la revendication 13, **caractérisé en ce que** la sortie de milieu de mise à température (84) se trouve en connexion avec un trop-plein (80) se trouvant dans la zone inférieure du boîtier de mise à température (60), qui est de préférence diamétralement opposée à l'entrée de milieu de mise à température (78).

15. Dispositif de culture selon la revendication 14, **caractérisé en ce que** les boîtiers de mise à température (60) des différents modules sont disposés en série, selon le courant concernant le milieu de mise à température.

16. Dispositif de culture selon l'une quelconque des revendications 1 à 15, **caractérisé en ce que** les récipients de culture (12) sont entourés d'un boîtier extérieur (52) pouvant être fermé de manière étanche.

17. Dispositif de culture selon la revendication 16, **caractérisé en ce que** le boîtier extérieur (52) est muni d'une sortie (128) pour un milieu gazeux.

18. Dispositif de culture selon l'une quelconque des revendications 1 à 17, **caractérisé en ce qu'**il consiste, à l'exclusion des détecteurs de niveau et des parties du dispositif d'alimentation, essentiellement en un matériau stérilisable, en particulier du verre et un matériau silicone.

19. Dispositif de culture selon l'une quelconque des revendications 1 à 18, **caractérisé en ce que** le dispositif d'alimentation (24 à 28) présente un dispositif de pompe travaillant de manière bidirectionnelle (24), en particulier une pompe péristaltique.

20. Dispositif de culture selon l'une quelconque des revendications 1 à 19, **caractérisé par** un réglage programmé (42), qui règle dans le temps un transmetteur de niveau (40).

21. Dispositif de culture selon la revendication 20, **caractérisé en ce que** le réglage programmé (42) du transmetteur de niveau est commuté périodiquement entre deux valeurs, dont une correspond à un niveau de liquide se trouvant en un intervalle donné au-dessus de la face supérieure de la surface de culture, et l'autre à un niveau se trouvant en un intervalle donné sous la face supérieure de la surface de culture.

22. Procédé de culture de cellules ou de composant tissulaires en utilisant un dispositif de culture selon l'une des revendications 1 à 21, **caractérisé en ce que** le niveau de liquide d'une solution nutritive pour les cellules ou parties tissulaires se trouve de manière alternée, pour un intervalle donné, au-dessus de la face supérieure d'une culture de cellules ou de parties tissulaires (culture immergée), et, pour un intervalle donné, sous la face supérieure de la culture (culture basale).

23. Procédé selon la revendication 22, **caractérisé en ce que** dans un intervalle de temps, au cours duquel le niveau de liquide se trouve sous la face supérieure de la culture, la culture est exposée à des substances solides, gazeuses ou sous forme d'aérosol.

24. Procédé selon la revendication 22 ou 23, **caractérisé en ce que** la position du niveau du liquide est réglée à l'aide d'un détecteur de niveau et d'un dispositif de pompe ainsi réglé.

25. Procédé selon l'une quelconque des revendications 22 à 24, **caractérisé en ce qu'**un ajustement temporel de la position du niveau du liquide est réalisé à l'aide d'un réglage programmé.
